# EUROPEAN PATENT APPLICATION

(11) **EP 1 527 739 A2**
(43) Date of publication of application: **04.05.2005**
(21) Application number: 04021099.9
(22) Date of filing: 06.09.2004
(51) Int. Cl.: A61B 10/00

(54) **Removable cannula instrument for taking biopsy samples**

(30) Priority: 05.09.2003 IT BO20030516
(71) Applicant: Avaltroni, Paolo, 46100 Mantova (IT)
(72) Inventor: Avaltroni, Paolo, 46100 Mantova (IT)
(74) Representative: Dall'Olio, Giancarlo

(57) **Abstract**

The needle instrument for taking biopsic samples comprises a cannula comprising a needle device (100) which comprises a body (1), a sliding gear (10), longitudinally sliding inside said body (1), a mandrel (5), longitudinally arranged in said body (1) and coming out from the distal end (1f) thereof and also slidingly inserted in a longitudinal through bore (11) made in said sliding gear (10), and a mandrel handle (7), fixed at the proximal end of said mandrel (5) and fit to make the mandrel handling easier.

The instrument is also provided with locking means (9), fit to engage said gear (10) and move it from said extended position to one of said loading, retracted positions.

A hollow cannula (50) is removably inserted in said needle device (100), coaxially and externally with respect to said mandrel (5), also engaged in a front portion (12) of said gear (10) and slidable together with it with respect to said body (1) and mandrel (5).

Locking means (30) are placed in said gear (10) and they are fit to removably lock corresponding complementary locking means (52) of said cannula (50) to the same gear (10).

## Description

The present invention fits into the technical sector relating to the manufacturing of instruments for taking biopsy samples, that is for collecting of biological tissue samples from a patient for analysing purposes.

More particularly, the present invention relates to a needle instrument, of the kind known as "partially automatic" one. This kind of device is fit to perform, once it has been manually inserted in the operating site, and then loaded by an operator, the remaining operations relative to the separation of the biopsic in a substantially automatic mode.

Several different kinds of needle instruments for collecting biopsic sample are so far known. These normally comprise a hollow cannula, which is inserted into a patient's organ or neoformation wherefrom a sample has to be taken. This latter is held inside the cannula using different known techniques, depending on different kind of instruments used. Once the sample has been entrapped, the cannula is then extracted together with the sample.

Very often inserting the cannula is made easier by a mandrel, slidable inside the same cannula, whose distal end protrudes from the cannula distal end. This allows the operating area to be reached without putting into the cannula hollow patient's tissues different from those belonging to the desired sample.

A kind of a commonly used biopsic device, particularly suitable to perform biopsy operations on soft tissues, is the so called "guillotine type" device. Said device comprises a longitudinal groove near the mandrel distal end. While the needle is being inserted into the patient's body, said groove rests inside the cannula. When the needle point reaches the sampling area, the mandrel is pushed forward, putting the longitudinal groove into contact with the surrounding tissue and thus filling it with said tissue. The cannula is then suddenly pushed forward by a known elastic means, and cuts the tissue portion contained in the longitudinal groove with a "guillotine" effect. Both cannula and mandrel are subsequently removed together, bringing the cropped sample with them.

In order to allow the aforesaid operating steps to be performed in a safe and repeatable way, thus avoiding those errors which could lead to sample losses or to collect samples of short size, there are known needle devices which perform the final operating steps automatically. Substantially, in said devices the needle, which comprises the cannula and its corresponding mandrel, is inserted into the patient's body by a specialist manually, under echographic or X-ray control. Once the cannula distal end and the mandrel point reach the sampling area, the needle is operated by the automatic operating device.

In a known embodiment of the aforesaid automatic device, this latter comprises an inner sliding gear. The proximal end of the needle cannula is fixed to said sliding gear. The proximal end of the mandrel is fixed to a handle, which is placed on the rear side of the automatic device. The mandrel is also slidably arranged in a longitudinal hole provided in the sliding gear, wherefrom it comes out inside the cannula and coaxially with the same. A spring which surrounds the mandrel coaxially is provided between the handle and the rear end of the sliding gear. A toothed bar is moreover associated with the handle, and acts on the sliding gear in order to load it, as the handle is pulled backwards against the elastic reaction of the spring.

There is moreover provided means for release the sliding gear at a predefined moment, e.g. by manually pushing the handle and the mandrel fixed thereto.

The above known automatic device is operated by loading the sliding gear first, pulling back the handle and the toothed bar, until the same gear reaches one of several possible starting positions. These starting positions correspond to different depth values for performing the biopsy operation.

The specialist inserts the distal portion of the needle into the patient's body, until the needle point reaches the sampling site. A subsequent manual forwarding of the handle, and of the mandrel fixed thereto, allows the specialist to push the distal portion of the mandrel into the sampling site until the set-up depth is reached, and then to free the sliding gear. This latter, pushed by the elastic action of the pre-loaded spring, leads the cannula forward, which completes the sample cutting and enclosing operation. The cannula-mandrel assembly is then removed by the patient's body, and the biopsic sample to be analysed is withdrawn from inside the cannula.

The automatic devices for taking biopsic samples of the kind described above are easy to use, effective and sufficiently reliable, but they have some operating limitations and drawbacks. Even when two or more sampling operations have to be performed in the same site or in sites situated close to the first one, the cannula-mandrel assembly must be removed from the patient's body after each operation, and it must be inserted again by means of a new puncture.

Moreover, if a single sampling operation is not successful and the taken sample is deemed not suitable for a further analysis, e.g. because it is too small, the needle instrument must be inserted again in the patient's body with a new puncture.

As the biopsic sampling operations are quite painful for the patients, a great relief could be given them if it would not be necessary to insert the needle instruments into their body for each sample to be taken, at least for the above described situations.

The main object of the present invention is to provide a needle instrument for taking biopsic samples which is able to allow multiple samples to be taken, at sampling sites close one each other, with a single insertion of the needle in the patient body.

A further object of the present invention is to provide a biopsy instrument able to achieve the above performance with substantially no increase in the instrument complexity, cost and reliability with respect to the automatic biopsy device of the known art.

The aforesaid objects are fully attained by a needle instrument for taking biopsic samples of soft tissues according to the independent claims.

### BRIEF DESCRIPTION OF THE DRAWINGS

The characteristic features of the present invention, as they will result from the appended claims, will appear in the following detailed description, made with reference to the attached drawing tables, wherein:
- figure 1 shows schematically a top view of a needle instrument for taking biopsic samples made according to the present invention, without its corresponding cannula;
- figure 2 shows a side sectional view of a cannula belonging to the needle instrument of figure 1;
- figure 3 shows a rear view of the cannula of figure 2;
- figure 4 shows a side view of the needle instrument of figure 1;
- figure 5 shows the needle instrument of figure 4 during the cannula mounting phase;
- figure 6 shows a partial, side sectional view at an enlarged scale, of the needle instrument of figure 1, with the cannula fully mounted therein.

### DESCRIPTION OF A PREFERED EMBODIMENT OF THE INVENTION

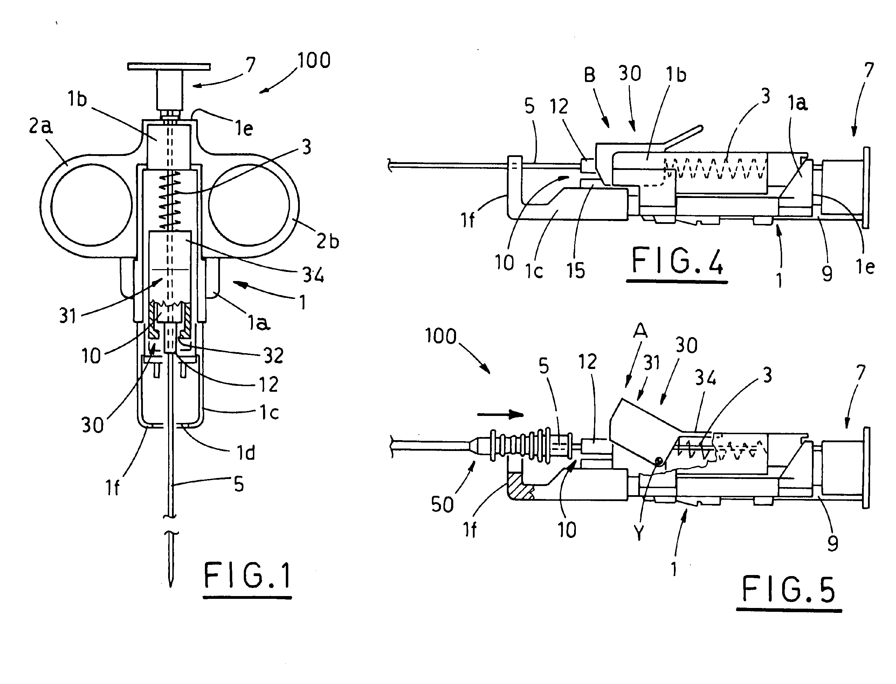

With reference to figure 1, numeral 100 indicates a needle device, belonging to a needle instrument for performing tissue biopsies made according to the present invention. It is particularly suited for taking biopsic samples, mainly of soft tissues, from a patient's body, in a partially automatic mode.
The needle device 100 comprises a body 1, which is substantially symmetrical with respect to a longitudinal axis. The body 1 comprises, for known manufacturing and functional purposes, a base 1a, an upper shell 1b fixed in the upper part of said base 1a, and a front cradle 1b, fixed to the front part of the same base 1a. This latter is moreover provided with a pair of annular members 2a,2b, arranged at both sides of the same base 1a, fit to help handling the needle device 100.

A mandrel 5 is arranged along a longitudinal symmetry plane of the body 1. Mandrel 5 is substantially a thin needle, sharply pointed at its distal end and also provided, near its distal end, with a longitudinal groove fit to receive a biopsic sample. The mandrel 5 extends for the whole body 1 length, and comes out from the front end therefrom, at an opening 1d provided in the cradle 1c.

The mandrel proximal portion comes out from the rear end 1e of the body 1. A mandrel handle 7 is fixed to the mandrel proximal end, for allowing the same mandrel 5 to be handled by a specialist.

A sliding gear 10 is enclosed between the base 1a and the upper shell 1b of body 1. The sliding gear 10 is mounted longitudinally in the same body 1 and is fit to slide between an extended, sampling position and one or more retracted, loading positions. Each one of said loading positions, which are normally in the number of three, defines a different device penetrating length and, subsequently, a different sample length.

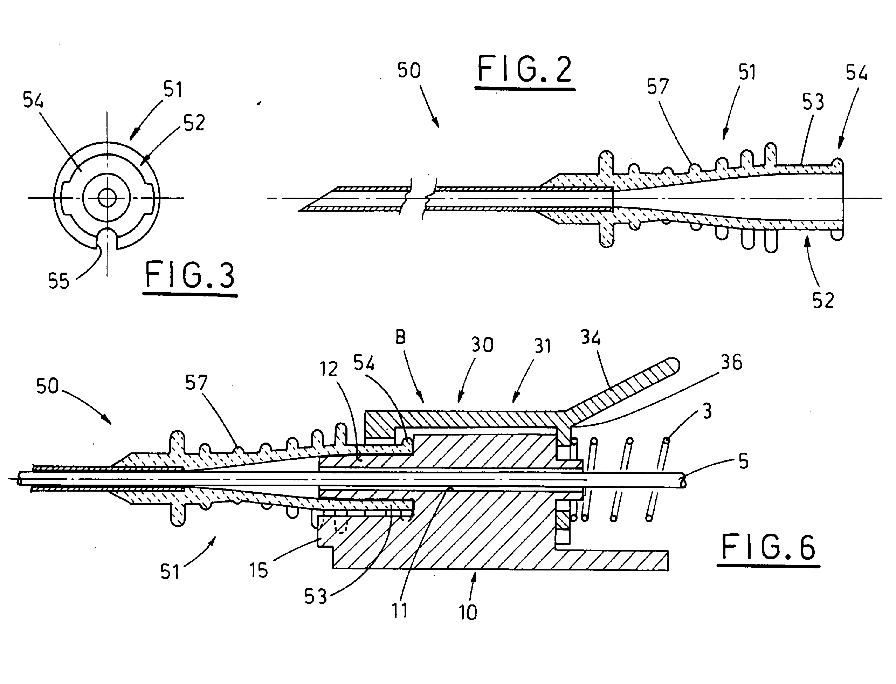

The sliding gear 10 is provided with a longitudinal through bore 11, fit to sliding receive the above mandrel 5 (see figures 1 and 6). In the rear portion of the sliding gear 10 there is engaged a launching spring 3, whose opposite end is engaged with an abutment made in the rear portion of the base 1a.

The launching spring 3 is coaxial with the mandrel 5, and surrounds a rear portion thereof. It is fit to exert an elastic compression against the sliding gear 10 when it slides toward the aforesaid retracted positions, and therefore to load the same gear 10 enough to allow it to go off to the extended position during the known sample taking operation.

The front portion 12 of the sliding gear 10, wherefrom the aforesaid through bore 11 comes out, has a substantially cylindrical shape. Under said front portion 12 there is provided a key 15, of elongated shape, which extend parallel with respect to the front portion 12.

The mandrel handle 7 is provided with gear locking means 9, which is only partially visible in the above figures, fit to lock the sliding gear 10 for drag it toward the loading, retracted positions. There is moreover provided complementary means for stopping the gear 10 in each one of its loading positions, and for releasing the same during the sample taking operations. All of these means are generally known in needle instruments of this type, and will not be described in depth in the following.

The needle instrument of the present invention moreover comprises a hollow cannula 50, (see also figures 1 and 2), suitable to be mounted on the sliding gear of the above described needle device 100, fit to slidably receive the mandrel 5. The cannula 50 comprises, at its proximal end, a hollow head 51, whose structure will be better detailed in the following.

In the sliding gear 10 there are also provided a locking member 30, fit to removably fix said cannula 50 to the sliding gear 10. The locking member 30 comprises a shell 31, placed in the upper part of the sliding gear 10 and surrounding the same gear. The shell 31 is articulated so that it can rotate on a transverse axis Y, in the lower part of the gear 10. In this way it can swing between a releasing position A, wherein it frees the cannula 50 (see figure 5), and a locking position B, wherein it locks the same cannula 50 in a mounted position (see figures 4 and 6).

The shell 31 is moreover provided, at its front end, with an opening 32 (see figure 1), shaped as the head 51 of cannula 50, when this latter is held by the same shell 31 in cooperation with the front portion 12 of the sliding gear 10. The shell 31 is normally held in its locking position B by a suitable elastic member. Particularly, but not exclusively, in the described embodiment of the present invention, said elastic member is the aforementioned launching spring 3, but used in a different operating mode with respect to its main loading operating mode.

As it can be appreciated in figure 6, an upper part of the launching spring 3 is arranged into contact with the upper, inner wall 36 of the shell 31. This upper wall is fit to act on the launching spring 3 by means of a cross bending of the same spring, thus receiving a corresponding elastic force.

The shell 31 moreover comprises a wing 34, placed in the upper rear part of the same shell, which extends slantwise in an upper direction with a predefined angle. The wing 31 is fit to allow the shell 31 to be operated manually from its locking position A to its releasing position B.

The cannula head 51 is provided, at its free end, with complementary locking means 52 (see figures 2,3 and 6), which consists of a cylindrical portion 53 and an annular thick rim 54. These cylindrical portion 53 and annular rim 54 are suitable to be engaged by the aforesaid front portion 12 of sliding gear 10 and front part of shell 31.

The head 51 also comprises gripping means 57, fit to make the cannula 50 handling by a specialist easier. This gripping means 57 comprises a plurality of rings of growing diameter peripherally made on the outer surface of the head 51.

In the lower part of the head 51 there is also provided a longitudinal groove 55, fit to engage the gear key 15 for defining the position of insertion for the cannula 50.

As far as it regards the preparing and practical performing of the sampling operations, the needle instrument operating steps are well known, and will not be detailed in the following.

As far as it regards the cannula 50, it is mounted on the needle device 100 before its use begins. The mandrel 5 is inserted in the cannula head 51, until this latter approaches the front part of the sliding gear 10. The shell 31 is then manually raised from its locking position B to its releasing position A, by operating on the wing 34 against the bending reaction of the launching spring 3. The cannula head 51 is then moved toward the gear 10, until its cylindrical portion 53 and its annular rim 54 engage the front portion 12 of said gear 10. At this point the shell 31 is moved back to its locking position B, until it encloses and locks the cannula head 51.

The cannula 50 releasing operation is as much as simple, because it is just necessary to act again on the wing 34 until the shell 31 reaches its releasing position A, then extract the mandrel 5, holding its taken biopsic sample inside its longitudinal groove 55, from the cannula 50, and finally moving back the shell again to its locking position B.

In this way, the biopsic sample could be removed from the patient's body, while the cannula 50 stays "in situ" for a further operation.

The above described biopsy instrument made according to the present invention advantageously allows the sample taking operations to become easier, faster and less painful, mainly in the case where multiple sampling operations have to be performed on the same site, or where the sample taken from a site would not be satisfactory and a second one must be taken.

The aforesaid biopsy instrument does not have substantially manufacturing complexity or expensive parts or materials to use. It is also reliable and cost effective.

## Claims

1. Needle instrument for taking biopsic samples, comprising a needle device (100) which comprises: a body (1); a sliding gear (10), longitudinally sliding inside said body (1) between an extended position and at least one retracted, loading position, against the action of an elastic launching member (5), fit to push said sliding gear (10) toward said extended position; a mandrel (5), longitudinally arranged in said body (1) and coming out from the distal end (1f) thereof and also slidingly inserted in a longitudinal through bore (11) made in said sliding gear (10); a mandrel handle (7), fixed at the proximal end of said mandrel (5) and fit to make the mandrel handling easier; locking means (9) for said sliding gear (10), fit to engage said gear (10) and move it from said extended position to one of said loading, retracted positions; said needle instrument being **characterised in that** it also comprises: a cannula (50), removably inserted in said needle device (100), coaxially and externally with respect to said mandrel (5), also engaged in a front portion (12) of said gear (10) and slidable together with it with respect to said body (1) and mandrel (5); locking means (30) placed in said gear (10) and fit to removably lock corresponding complementary locking means (52) of said cannula (50) to the same gear (10).

2. Needle device for taking biopsic samples comprising: a body (1); a sliding gear (10), longitudinally sliding in said body (1) from an extended position and at least one retracted, loading position, against the action of an elastic launching member (5), fit to push said sliding gear (10) toward said extended position; a mandrel (5), longitudinally arranged in said body (1) and coming out from the distal end (1f) thereof and also slidingly inserted in a longitudinal through bore (11) made in said sliding gear (10); a mandrel handle (7), fixed at the proximal end of said mandrel (5) and fit to make the mandrel handling easier; locking means (9) for said sliding gear (10), fit to engage said gear (10) and move it from said extended position to one of said loading, retracted positions; said needle device (100) being **characterised in that** it moreover comprises: locking means (30) arranged in said sliding gear (10) and fit to removably lock complementary locking means (52) of a cannula (50) to said sliding gear (10), said cannula (50) being inserted in said needle device (100) coaxially and externally with respect to said mandrel (5), being also engaged in a front portion (12) of said gear (10) and sliding with this latter with respect to said body (1) and mandrel (5).

3. Needle device according to Claim 1, **characterised in that** said locking member (30) comprises a shell (31), arranged in the upper part of said sliding gear (10) and surrounding at least partially said gear upper part, said shell (31) being articulated on a transverse axis Y for swinging between a release position A, wherein it releases said cannula (50), and a locking position B, wherein it locks said cannula (50), said shell (31) being also provided, at its front end, with an opening (32) fit to receive and retain complementary locking means (52) of said cannula (50) when this latter is engaged in the front portion (12) of said gear (10), said shell (31) being normally held in its locking position B by suitable elastic means.

4. Needle device according to Claim 1 or to Claim 2, **characterised in that** said elastic means coincide with said launching elastic means (3) of said gear (10), and comprises a spring (3) arranged coaxially with respect to said mandrel (5) at the proximal portion thereof which comes out from said gear (10), said spring (3) being operated according to a transversal bending action by the upper, inner wall (36) of said shell (31).

5. Needle device according to Claim 2, **characterised in that** said shell (31) moreover comprises a wing (34), placed in the upper, rear portion of said shell (31), extending slantwise in an upper direction with a predefined raising angle and fit to make the swinging operation of the same shell (31) from its locking position A to its releasing position B easier.

6. Needle device according to Claim 2, **characterised in that** said sliding gear (10) comprises a key (15), provided in the front part thereof, under said front portion (12) of the same gear (10), fit to engage a corresponding longitudinal groove (55) made in a head (51) of said cannula (50) for defining the inserting direction for said cannula (50) in said needle device (100).

7. A cannula for a needle instrument for taking biopsic samples, usable with a needle device (100) of the kind comprising: a sliding gear (10), longitudinally sliding inside said body (1) between an extended position and at least one retracted, loading position, against the action of an elastic launching member (5), fit to push said sliding gear (10) toward said extended position; a mandrel (5), longitudinally arranged in said body (1) and coming out from the distal end (1f) thereof and also slidingly inserted in a longitudinal through bore (11) made in said sliding gear (10), fit to be inserted, at least for a distal portion thereof, in said cannula (50); this latter being **characterised in that** it comprises a hollow head (51) mounted at the proximal end of said cannula (50), said head (51) being provided with complementary locking means (52), fit to engage corresponding locking means (30) of said needle device (100).

8. Cannula according to Claim 7, **characterised in that** said complementary locking means (52) comprises a cylindrical portion (53) and a thick annular rim (54).

9. Cannula according to Claim 7, **characterised in that** said head (51) moreover comprises a longitudinal groove (55), provided on said complementary locking means (52) and fit to engage a corresponding key (15) provided in the front part of said sliding gear (10), for defining the direction of insertion for said cannula (50) in said needle device (100).

10. Cannula according to Claim 7, **characterised in that** it also comprises gripping means (57), peripherally arranged in said head (51) and fit to make the cannula (50) handling easier.
